(19) Europäisches Patentamt
European Patent Office
Office européen des brevets

(11) **EP 2 550 095 B1**

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(51) Int Cl.:
***B01J 20/10*** *(2006.01)*     ***B01J 21/08*** *(2006.01)*
***C01B 33/193*** *(2006.01)*     ***C09C 1/30*** *(2006.01)*

(21) Anmeldenummer: **11707879.0**

(22) Anmeldetag: **14.03.2011**

(86) Internationale Anmeldenummer:
**PCT/EP2011/053798**

(87) Internationale Veröffentlichungsnummer:
**WO 2011/117100 (29.09.2011 Gazette 2011/39)**

(54) **GROBTEILIGE TRÄGERKIESELSÄUREN**

COARSE SUPPORT SILICA PARTICLES

ACIDES SILICIQUES SUPPORTS À PARTICULES GROSSIÈRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.03.2010 DE 102010003204**

(43) Veröffentlichungstag der Anmeldung:
**30.01.2013 Patentblatt 2013/05**

(73) Patentinhaber: **Evonik Degussa GmbH**
**45128 Essen (DE)**

(72) Erfinder:
• **DREXEL, Claus-Peter**
**63263 Neu-Isenburg (DE)**
• **HASELHUHN, Frank**
**63456 Hanau (DE)**
• **HEINDL, Frank**
**63517 Rodenbach (DE)**
• **RAUSCH, Ralf**
**63776 Mömbris (DE)**
• **STEIN, Günter**
**61130 Nidderau 4 (DE)**

(56) Entgegenhaltungen:
**EP-A1- 0 725 037    EP-A1- 0 937 755**
**WO-A2-01/64168**

EP 2 550 095 B1

**Beschreibung**

Die vorliegende Erfindung betrifft neuartige granuläre Kieselsäuren für den Einsatz als Trägermaterial, insbesondere als Träger für Katalysatoren in verschiedenen Reaktorsystemen, deren Herstellung und deren Verwendung.

In vielen Anwendungsgebieten wie z.B. im Bereich der Erzeugnisse für den Pflanzenschutz, bei pharmazeutischen Wirkstoffen, bei der Herstellung von Futtermitteln und Futtermitteladditiven oder in der Lebensmittelindustrie werden Trägermaterialien eingesetzt um z.B. flüssige oder harzförmige Wirkstoffe in eine fließfähige und lagerstabile Form zu überführen. Zur Herstellung der festen Formulierungen werden flüssige oder schmelzbare Stoffe oder Stoffgemische teilweise zusammen mit Hilfsstoffen (zum Beispiel Tensiden und Sprengmitteln) auf Trägermaterialien aufgezogen. Bei festen Stoffen dienen die Trägermaterialien dabei in erster Linie als Füllstoff, während sie bei flüssigen oder niedrig schmelzenden Stoffen die Flüssigkeit aufnehmen. Dadurch sollen leicht handhabbare, äußerlich trockene Absorbate erhalten werden, die dann bspw. im Agrar-Bereich als Pulver direkt (sogenannte WP, wettable Powder) oder, weiterverarbeitet, als Granulate/Extrudate (WG, Water dispersible Granules) auf den Markt kommen. Falls es die jeweiligen Anwendungen erfordern, können die absorbierten Lösungen getrocknet werden oder die absorbierten Schmelzen zur Erstarrung gebracht werden. Im Allgemeinen wird von beladenen Trägermaterialien gesprochen.

Eine wesentliche Anforderung an das Trägermaterial ist eine hinreichend hohe Saugfähigkeit, so dass möglichst wenig Trägermaterial eingesetzt werden muss. Daher beschäftigen sich eine Reihe von Patentanmeldungen, wie z.B. die DE102006002765, mit Verfahren zur Steigerung des Gehalts an absorbiertem Material auf dem Trägermaterial. Diese Verfahren sind jedoch sehr aufwendig durchzuführen und haben sich bislang nicht großtechnisch durchgesetzt.

Eine weitere Anforderung an das Trägermaterial ist, dass die Absorbate eine gute Fließfähigkeit und somit gute Verarbeitbarkeit aufweisen. Ferner sollen die Kieselsäuren beim Transport, Umfüllen und der Herstellung der Absorbate möglichst wenig stauben. Zur Verbesserung der Fließfähigkeit wurde daher z.B. in der EP 0984772 B1 und der EP 0966207 B1 vorgeschlagen mikrogranuläre Kieselsäuren mit in etwa sphärischer Form und mit einer mittleren Partikelgröße von über 150 $\mu$m als Trägermaterial zu verwenden. Die auf diese Weise erhaltenen Absorbate weisen zwar eine verbesserte Fließfähigkeit auf, die Verarbeitungseigenschaften der Kieselsäuren sind jedoch nicht optimal, da bei der Herstellung von Absorbaten mit diesen Kieselsäuren häufig Anbackungen in den Mischern beobachtet wurden, welche teilweise aufwendig entfernt werden müssen.

Im Bereich der Festbettkatalyse kommen weitere Anforderungen an das Trägermaterial hinzu. So muss z.B. sichergestellt werden, dass es bei Reaktionen in Festbettreaktoren, bei denen die Reaktanden einen mit beladenen Trägermaterialien, auf denen ein Katalysator aufgebracht ist, gefüllten Reaktionsraum durchströmen, zu möglichst geringen Druckverlusten im Reaktionsraum kommt. Bei Reaktionen, bei denen ein mit Katalysator beladenes Trägermaterial in einem Reaktionsmedium suspendiert wird, muss das Trägermaterial am Ende der Reaktion leicht wieder abgetrennt werden können. Schließlich fordern Reaktionen in einem Fließbettreaktor, dass die beladenen Trägermaterialien dort gut fluidisiert werden können. Es ist somit offensichtlich, dass unterschiedliche Reaktortypen ganz unterschiedliche Anforderungen an die beladenen Träger und somit auch an das Trägermaterial stellen. Bislang fokussierten sich die Forschungsarbeiten wie oben gezeigt weitgehend auf die Absorbtionsfähigkeit von Trägermaterialien bzw. auf deren Verarbeitbarkeit. Für die speziellen Anforderungen bei katalytischen Verfahren gibt es noch keine zufriedenstellenden Trägermaterialien.

Es besteht daher nach wie vor ein hoher Bedarf an preiswerten Trägermaterialen welche gut verarbeitbar sind, es erlauben hoch beladene und gut fließfähige Absorbate herzustellen und zudem gut für katalytische Verfahren geeignet sind.

Unter Absorbaten werden mit einem Aktivstoff oder einem Aktivstoffgemisch direkt oder in Dispersion, Lösung oder Schmelze beladene Trägerkieselsäuren verstanden. Zusätzlich zu dem Aktivstoff oder Aktivstoffgemisch können noch Hilfsstoffe beladen werden. Die auf die Trägerkieselsäure beladenen Stoffe werden hier auch als absorbierte Substanzen bezeichnet.

Aufgabe der vorliegenden Erfindung war es daher, neuartige granuläre Kieselsäuren bereitzustellen, welche zumindest einige der Nachteile der Trägermaterialien des Standes der Technik nicht oder nur in verringertem Maße aufweisen und welche es erlauben, neuartige beladene Träger mit verbesserten anwendungstechnischen Eigenschaften herzustellen. Ferner soll ein Verfahren zur Herstellung dieser granulären Kieselsäuren und zur Herstellung der beladenen Träger bereitgestellt werden.

Eine spezielle Aufgabe bestand darin, granuläre Kieselsäuren bereitzustellen, welche es erlauben, mit Katalysatoren beladene Träger herzustellen, die sehr leicht aus Suspensionen abgetrennt werden können.

Eine weitere spezielle Aufgabe bestand darin, granuläre Kieselsäuren bereitzustellen, welche es erlauben, mit Katalysatoren beladene Träger herzustellen, die in Festbettreaktoren möglichst wenig Druckverlust des Durchströmenden Reaktionsgemisches verursachen.

Diese und weitere nicht explizit genannte Aufgaben werden durch die in den Ansprüchen, der Beschreibung und den Beispielen näher definierten granulären Kieselsäuren, beladenen Trägern und Herstellungsverfahren gelöst.

Überraschender Weise wurde gefunden, dass es nicht ausreicht Trägermaterialien mit sphärischer Form und

EP 2 550 095 B1

einer mittleren Partikelgröße von über 150 $\mu$m, wie in der EP 0984772 B1 und der EP 0966207 B1 beschrieben, zur Herstellung von Absorbaten zu verwenden, da mit solchen Trägermaterialien in Festbettreaktionen nicht zufriedenstellende Ergebnisse erzielt wurden.

Die Erfinder haben durch eingehende Untersuchungen herausgefunden, dass ein wesentliches Kriterium, das das Trägermaterial erfüllen muss, dessen Stabilität gegenüber mechanischen Belastungen ist. Ist das Trägermaterial zu weich, so kann es bei der Beladung des Trägermaterials im Mischer zu Anbackungen kommen. Weiterhin kann es bei der Weiterverarbeitung der beladenen Trägermaterialien und während der Befüllung der Reaktoren dazu kommen, dass ein Teil der Trägermaterialien pulverisiert wird und das so gebildete Pulver den Reaktor verstopft, so dass es zu einem erhöhten Druckverlust im Reaktor kommt.

Es ist weiterhin bekannt, dass es auch beim Überströmen eines Fluids über die beladenen Träger im Reaktor zu Abriebserscheinungen kommen kann, wenn die Trägermaterialien nicht hinreichend hart sind, dadurch können offensichtlich Druckverluste im laufenden Betrieb von Festbettreaktoren bzw. Filtrationsprobleme im Bereich der suspendierten Katalysatoren erklärt werden.

Schließlich wurde herausgefunden, dass die Partikel der Trägermaterialien eine spezielle Korngrößenverteilung haben müssen, um einen möglichst geringen Strömungswiderstand im Festbettreaktor bzw. im Wirbelschichtreaktor hervorzurufen.

Durch ein spezielles Herstellungsverfahren gelang es, die Härte der erfindungsgemäßen granulären Kieselsäuren so zu steigern, dass diese der mechanischen Belastung bei der Absorbatherstellung, der Absorbatverarbeitung und dem Betrieb von Reaktoren deutlich besser standhalten und somit deutlich verbesserte anwendungstechnische Eigenschaften aufweisen. Weiterhin wurde die Partikelgrößenverteilung verbessert und sichergestellt, dass die gehärteten granulären Kieselsäuren eine hinreichend hohe Porosität aufweisen. Die erfindungsgemäßen granulären Kieselsäuren zeichnen sich daher durch eine hinreichend hohe Porosität - ausgedrückt durch das Hg-Porenvolumen - besser stabilisierte Porenwände - ausgedrückt durch das Verhältnis der mittleren Partikelgröße ($d_{50}$-Wert) ohne Ultraschalleinwirkung zur mittleren Partikelgröße ($d_{50U}$-Wert) nach 3 min Ultraschalleinwirkung - sowie eine optimierte Partikelgröße - ausgedrückt durch den $d_{Q3=10\%}$-Wert zur Beschreibung des Feinanteils sowie durch den $d_{Q3=90\%}$-Wert zur Beschreibung des Grobanteils - aus. D.h. die Erhöhung der mechanischen Stabilität konnte erreicht und gleichzeitig eine hohe Absorptionsfähigkeit bei optimaler Partikelgrößenverteilung erhalten werden.

In einer bevorzugten Ausführungsform weisen die erfindungsgemäßen Kieselsäuren einen annähernd neutralen pH-Wert auf, so dass diese sehr universell als Träger eingesetzt werden können und keine negativen Auswirkungen auf die Lagerstabilität der absorbierten Materialien aufweisen.

Weiterhin weisen die erfindungsgemäßen Kieselsäuren im vergleich zu kommerziell eingesetzten Trägerkieselsäuren wie z.B. Zeosil 165 GR der Firma Rhodia Chimie oder Zeodent DP-9175 der Firma Huber Corp. ein optimales Verhältnis aus Härte, d.h. mechanischer Stabilität, der Partikelgrößenverteilung bezüglich Feinanteil und Grobanteil sowie Saugvermögen auf.

Gegenstand der vorliegenden Erfindung sind daher granuläre Kieselsäuren mit

- einem Hg-Porenvolumen (< 4 $\mu$m) von mehr als 0,90 ml/g
- einem $d_{Q3=10\%}$-Wert von mehr als 400 $\mu$m bei gleichzeitig einem $d_{Q3=90\%}$-Wert von weniger als 3000 $\mu$m, wobei die Granulate durch Siebung oder Siebgranulation bei einer Siebgröße von 3000 $\mu$m und Absiebung des Feinanteils mit einer Siebmaschenweite von 400 $\mu$m auf die entsprechende Partikelfraktion eingestellt wird,
- einem Verhältnis des $d_{50}$-Werts ohne Ultraschalleinwirkung zu $d_{50}$-Wert nach 3 min Ultraschalleinwirkung von < 4,00 aufweist, wobei die Messung an einer Fraktion von Partikeln von 400 bis 500 $\mu$m erfolgt.

Weiterhin Gegenstand der vorliegenden Erfindung sind granuläre Kieselsäuren, die neben den o. g. Parametern zumindest eine der nachfolgenden Eigenschaften aufweisen:

- pH-Wert von 5 bis 8,5
- ein Verhältnis des $d_{50}$-Werts ohne Ultraschalleinwirkung zu $d_{50}$-Wert nach 3 min Ultraschalleinwirkung von 1,00 bis 3,00, bevorzugt 1,00 bis 2,60, besonders bevorzugt 1,00 bis 2,10, speziell bevorzugt 1,00 bis 1,60 aufweist. Die Messung erfolgt dabei an einer Fraktion von Partikeln von 400 bis 500 $\mu$m.

Gegenstand der vorliegenden Erfindung ist zudem ein erstes Verfahren zur Herstellung der erfindungsgemäßen granulären Kieselsäuren umfassend die Schritte

a) Bereitstellen einer gefällten oder pyrogenen Kieselsäure, getrocknet und/oder vermahlen mit

- einer mittleren Partikelgröße $d_{50}$ ohne Ultraschallbehandlung von 0,1 bis 350 $\mu$m,
- einer BET-Oberfläche von 30 bis 800 m$^2$/g, und

- einer DBP-Zahl von 140 bis 400 g/100g;

b) Befeuchtung der Kieselsäure aus Schritt a) entsprechend des angewendeten Formgebungsverfahren auf einen Trocknungsverlust von 30-80 Gew.%

c) Formgebung der Kieselsäure aus Schritt b) durch Extrusion, Granulation, Kompaktierung, oder andere übliche Formgebungsverfahren;

d) Trocknung der Kieselsäureformkörper in dafür geeigneten Trocknungsaggregaten; und

e) Siebgranulation oder Siebung der Granulate bei einer Siebgröße von 3000 $\mu$m und Absiebung des Feinanteils mit einer Siebmaschenweite von 400 $\mu$m.

Alternativ zu dem zuvor beschriebenen ersten erfindungsgemäßen Verfahren kann auch ein wasserhaltiger Filterkuchen mit einem Trocknungsverlust von 30-80 Gew.% als Ausgangsmaterial für Schritt c) verwendet werden.

Weiterhin Gegenstand der vorliegenden Erfindung ist ein zweites Verfahren zur Herstellung der erfindungsgemäßen granulären Kieselsäuren umfassend die Schritte

i) Bereitstellen einer gefällten oder pyrogenen Kieselsäure, getrocknet und/oder vermahlen mit einem Trocknungsverlust < 30 Gew.%, und mit

- einer mittleren Partikelgröße $d_{50}$ ohne Ultraschallbehandlung von 0,1 bis 350 $\mu$m,
- einer BET-Oberfläche von 30 bis 800 m$^2$/g, und
- einer DBP-Zahl von 140 bis 400 g/100g;

ii) Formgebung der Kieselsäure aus Schritt i) durch Trockenkompaktierung, vorzugsweise zwischen zwei rotierenden Walzen, bei einem spezifischen Anpressdruck von 0,5 kN/cm Walzenbreite bis 12 kN/cm Walzenbreite zu Stülpen, und

iii) Siebgranulation oder Siebung der Stülpen bei einer Siebgröße von 3000 $\mu$m und Absiebung des Feinanteils mit einer Siebmaschenweite von 400 $\mu$m.

In allen oben beschriebenen erfindungsgemäßen Verfahren ist es möglich die Härte der Partikel weiter zu steigern, indem diese einer Behandlung mit Wasserdampf bei erhöhter Temperatur, wie beispielsweise 70°C bis 400°C, unterzogen werden. Im Anschluss ist eventuell ein weiterer Trocknungsschritt notwendig.

Weiterhin ist es möglich, die Härte der Partikel zu steigern, indem diese für eine gewisse Zeit mit einer alkalischen Substanz in Kontakt gebracht werden, um den pH Wert der Partikel anzuheben. Das Verfahren ist genauer in DE 102008035867 A1 beschrieben.

Eine weitere Möglichkeit, die Härte der Partikel zu steigern, besteht darin die Partikel bei einer erhöhten Temperatur, typischerweise zwischen 700°C und 1200°C, für eine gewisse Zeit (in der Regel < 1 h) zu calcinieren.

Die vorherbeschriebenen Verfahrensschritte zur Härtung der Partikel können vor oder nach dem Verfahrensschritt der Siebgranulation und Siebung durchgeführt werden.

Die Befeuchtungs- und/oder Granulierungsverfahrensschritte aus dem ersten erfindungsgemäßen Verfahren können in einem schnell laufenden intensiven Mischer, Kneter, Kompaktor, Tellergranulator und/oder Lochmatritzenpresse oder ähnlichem durchgeführt werden. Alternativ kann sich nach der Befeuchtung eine Extrusion anschließen oder es kann ein wasserhaltiger Filterkuchen direkt extrudiert werden. Extrudierte Formkörper können anschließend durch weitere geeignete Verfahren in ihrer geometrischen Form verändert werden (z.B. Spheronizer der Fa. Caleva).

Die Trocknungsverfahrensschritte aus dem ersten erfindungsgemäßen Verfahren können beispielsweise in Trockenschränken, Wirbelschichttrocknern, Bandtrocknern o.ä. durchgeführt werden. Die getrockneten Formkörper werden anschließend durch weitere Verfahren wie z.B. Siebung oder Siebgranulation bei einer Siebgröße von 3000 $\mu$m und Absiebung des Feinanteils mit einer Siebmaschenweite von 400 $\mu$m auf die entsprechende Partikelgrößenfraktion eingestellt.

Der Formgebungsschritt aus dem zweiten erfindungsgemäßen Verfahren wird vorzugsweise in einem Kompaktor z.B. in einem Apparat der Firma Hosokawa Bepex GmbH wie Bepex L200/50 oder der Firma Alexanderwerk AG durchgeführt.

Die Siebgranulation aus beiden erfindungsgemäßen Verfahren wird bevorzugt in Apparaten wie einer Siebmühle der Firma Frewitt oder der Firma Hosokawa Bepex GmbH durchgeführt. Die Siebung kann mittels aller bekannten Techniken erfolgen, vorzugsweise mittels eines Schwingsiebs von Firmen wie Vibra, Engelsmann oder Allgeier. Es können mehrere Siebe oder mehrere Siebungsschritte durchgeführt werden.

Gegenstand der vorliegenden Erfindung ist weiterhin die Verwendung der erfindungsgemäßen Kieselsäuren als Trägermaterial, bevorzugt für Katalysatoren.

Schließlich sind Gegenstand der vorliegenden Erfindung Absorbate umfassend zumindest eine erfindungsgemäße Kieselsäure.

Die Gegenstände der vorliegenden Erfindung werden nachfolgend im Detail beschrieben. Im Rahmen der vorliegenden Erfindung werden die Begriffe Kieselsäure(n), gefällte Kieselsäure(n) und pyrogene Kieselsäure(n) synonym verwendet.

Eine hinreichend hohe Porosität stellt sicher, dass die erfindungsgemäßen granulären Kieselsäuren ein ausreichendes Porenvolumen im Bereich der Meso- und/oder Makroporen besitzt und somit der Katalysator gut für die Reaktanden zugänglich ist und gleichzeitig möglichst wenig Trägermaterial zur Herstellung der erfindungsgemäßen Absorbate benötigt wird. Die erfindungsgemäßen granulären Kieselsäuren weisen daher ein Hg-Porenvolumen (< 4 $\mu$m) von mehr als 0,90 ml/g, bevorzugt von mehr als 1,35 ml/g, besonders bevorzugt mehr als 1,60, ganz besonders bevorzugt mehr als 1,80, speziell bevorzugt mehr als 1,90 auf.

Weiterhin bevorzugte erfindungsgemäße granuläre Kieselsäuren weisen ein Hg-Porenvolumen (<4$\mu$m) von 0,9 bis 1,34 ml/g, besonders bevorzugt von 0,9 ml/g bis 1,30 ml/g, ganz besonders bevorzugt von 0,9 ml/g bis 1,20 ml/g auf.

Eine weitere wesentliche Eigenschaft der erfindungsgemäßen granulären Kieselsäuren ist ihre Härte. Ist die Porosität hoch, so kann es vorkommen, dass die mechanische Stabilität nicht mehr gewährleistet werden und es zu einer erhöhten Bildung von Feinanteil bei mechanischer Belastung der Kieselsäure bzw. der damit hergestellten Absorbate, kommen kann. Die mechanischen Belastungen bei der Verpackung und dem Transport der Kieselsäure, bei der Herstellung der Absorbate sowie bei der Verwendung der beladenen Trägermaterialien werden simuliert durch Einwirkung von Ultraschallwellen auf die in Wasser suspendierte Kieselsäure für 3 min. Das Verhältnis von $d_{50}$-Wert ohne Ultraschalleinwirkung zu $d_{50}$-Wert nach 3 min Ultraschalleinwirkung gibt Auskunft darüber, um wie viel sich der $d_{50}$-Wert durch die mechanische Belastung verringert hat. Je härter die Kieselsäure ist, desto geringer ist die Differenz zwischen $d_{50U}$-Wert nach Ultraschalleinwirkung und $d_{50}$-Wert ohne Ultraschalleinwirkung, d.h. im Idealfall wäre das Verhältnis von $d_{50}$-Wert ohne Ultraschalleinwirkung zu $d_{50U}$-Wert nach 3 min Ultraschalleinwirkung gleich 1,00. Die erfindungsgemäßen granulären Kieselsäuren weisen trotz ihrer großen mittleren Partikelgröße eine sehr gute Härte auf, so dass das Verhältnis von $d_{50U}$-Wert ohne Ultraschalleinwirkung zu $d_{50}$-Wert nach 3 min Ultraschalleinwirkung kleiner 3,00, bevorzugt kleiner 2,60, besonders bevorzugt kleiner 2,10 und speziell bevorzugt kleiner 1,60 ist. Die Messung erfolgt dabei an einer Fraktion von Partikeln von 400 $\mu$m - 500 $\mu$m.

Die Partikelgrößenverteilung - charakterisiert durch den $d_{Q3=10\%}$-Wert und den $d_{Q3=90}$-Wert - ist wichtig um gute Strömungseigenschaften in Festbettreaktoren bzw. um gute Fluidisierungseigenschaften in Wirbelbettreaktoren sicher zu stellen. Zu große Partikel weisen nicht genügend spezifische Oberfläche für die Reaktion, Lösung und Diffusion auf. Zu kleine Partikel wiederum erhöhen den Strömungswiderstand. Die erfindungsgemäßen granulären Kieselsäuren weisen daher einen $d_{Q3=10\%}$-Wert > 400 $\mu$m und $d_{Q3=90}$-Wert < 3000 $\mu$m auf.

Die erfindungsgemäßen granulären Kieselsäuren weisen bevorzugt einen pH-Wert im Bereich von 5 bis 8,5 auf. Dieser weitgehend neutrale pH-Wert der Kieselsäuren stellt ein breites Anwendungsspektrum im Hinblick auf die zu absorbierenden Flüssigkeiten sicher, da zu stark saure bzw. zu stark basische Trägermaterialen die Zersetzung oder anderweitige chemische Umwandlung der zu absorbierenden Flüssigkeiten auslösen bzw. beschleunigen können.

Für Trägeranwendungen ist eine Reihe von Kieselsäuren auf dem Markt, welche im erfindungsgemäßen Verfahren eingesetzt werden können. Beispiele hierfür sind die Kieselsäuren SIPERNAT® 50, SIPERNAT® 50S, 500LS, 22, SIPERNAT® 22S, SIPERNAT® 22 LS und SIPERNAT® 33 der Firma Evonik Degussa GmbH. Wie die Erfinder herausgefunden haben, sind diese Kieselsäuren - obwohl speziell für Trägeranwendungen entwickelt - selbst nicht geeignet oder nur unzureichend geeignet um als Trägermaterial im Bereich der katalytischen Verfahren eingesetzt zu werden. Ursache hierfür ist - speziell bei den sprühgetrockneten, düsenturmgetrockneten und/oder vermahlenen Partikeln - deren zu geringe Partikelgröße, welche wie zuvor geschildert zu einem unerwünschten Druckanstieg im Reaktor führen kann. Durch dass erfindungsgemäße Verfahren wird eine Kompaktierung dieser Kieselsäuren vorgenommen, wobei die Partikelgröße und die Festigkeit der dadurch erzeugten Partikel durch das erfindungsgemäße Verfahren derart gesteuert wird, dass Partikel mit einer optimalen Korngrößenverteilung und Härte erhalten werden, welche einen geringen Strömungswiderstand im Reaktor aufweisen bzw. leicht aus Suspensionen abfiltrierbar sind.

Neben den bereits genannten Kieselsäuren können in Schritt a) des erfindungsgemäßen ersten Verfahrens beispielsweise die Kieselsäuren SIPERNAT® 2200, Aerosil® 200 der Firma Evonik Degussa GmbH, Tixosil® 38 A bis X der Firma Rhodia Chimie, HiSil® SC 60 und HiSil® SC 72 der Firma PPG, Hubersil® 5170 der Firma Huber sowie die in den Europäischen Patenten EP 0984772 B1, EP 0966207 B1 und EP 0937755 A1 offenbarten Kieselsäuren verwendet werden.

Die im erfindungsgemäßen Verfahren eingesetzten Kieselsäuren weisen

- eine mittlere Partikelgröße $d_{50}$ ohne Ultraschallbehandlung von 0,1 bis 350 $\mu$m, bevorzugt von 0,1 bis 200 $\mu$m, besonders bevorzugt von 0,1 bis 150 $\mu$m und ganz besonders bevorzugt von 1 bis 50 $\mu$m;
- eine BET-Oberfläche von 30 bis 800 m$^2$/g, bevorzugt von 40 bis 700 m$^2$/g, besonders bevorzugt von 50 bis 600 m$^2$/g, ganz besonders bevorzugt von 150 bis 550 m$^2$/g;
- eine DBP-Zahl von 140 bis 400 g/(100g), bevorzugt von 140 bis 350 g/(100g), besonders bevorzugt von 190 bis 350 g/(100g), ganz besonders bevorzugt von 290 bis 350 g/(100g)

auf.

Das erfindungsgemäße erste Verfahren wird vorzugsweise in einem Mischer, Kneter oder Kompaktor (optional mit nachgeschaltetem Extruder) und nachgeschaltetem Trockner, Siebgranulator und Sieb durchgeführt. Beispielsweise kann zunächst die vorgelegte Kieselsäure z.B. in einem Apparat der Firma Eirich GmbH mit Flüssigkeit benetzt werden (sofern nicht Filterkuchen direkt zum Einsatz gelangt), anschließend verdichtet bzw. kompaktiert, danach extrudiert und getrocknet. Ebenfalls ist es möglich, die mit Flüssigkeit benetzte und verdichtete bzw. kompaktierte Kieselsäure zu trocknen, anschließend eine Siebgranulation durchzuführen und danach auf die gewünschte Kornfraktion zu sieben.

Die Härte der finalen Trägerpartikel kann durch das Maß der Verdichtung bzw. Kompaktierung der Ausgangskieselsäure gesteuert werden. Die Verdichtung erfolgt in der Regel durch den Zusatz von Wasser bei gleichzeitigem Eintrag von Scherenergie. Weiterhin können auch wässrige Lösungen wie Celluloselösungen oder Öle zugegeben werden, die geeignet sind, als Binder zwischen den Partikeln zu fungieren. Die Flüssigkeit wird, bezogen auf eine Dichte von 1,00 g/mL, vorzugsweise in Anteilen von 50 bis 90 Gew.%, besonders bevorzugt in Anteilen von 60 bis 90 Gew.% und ganz besonders bevorzugt in Anteilen von 65 bis 90 Gew.% zugegeben. Des Weiteren kann während der Kompaktierung ein Feststoff, der geeignet ist, als Binder zwischen den Partikeln zu fungieren, wie beispielsweise Cellulose, Wachse oder Polymere oder Monomere welche anschließend polymerisiert werden zugegeben werden. Der Feststoff wird in Anteilen von 0,1 bis 50 Gew.%, bevorzugt in Anteilen von 0,5 bis 15 Gew.%, besonders bevorzugt in Anteilen zwischen 0,5 und 8 Gew.% zugegeben.

In einer bevorzugten Ausführungsform werden die Trägermaterialien ohne die Zuführung von Bindern verdichtet bzw. kompaktiert.

Die Kompaktierung wird vorzugsweise bei einer Temperatur von 10°C bis 90°C, besonders bevorzugt von 10°C bis 70°C durchgeführt.

Die Formgebung im ersten erfindungsgemäßen Verfahren erfolgt bevorzugt derart, dass die Ausgangskieselsäure in der Mischeinheit unter Zuhilfenahme der zugesetzten Flüssigkeit solange intensiv verdichtet wird, bis es zum partiellen Flüssigkeitsaustritt kommt und die Granulation der Partikel einsetzt. Die so erhaltenen Granulate (Rohgranulate) können durch einen Extrusionsschritt in ihrer Partikelgröße vereinheitlicht und anschließend getrocknet werden. Des weiteren können die feuchten Rohgranulate, bei Weglassung des Extrusionsschritts, auch direkt getrocknet und durch ein Sieb mit einer charakteristischen Größe von 3000 μm passiert werden, wobei die Partikel, die größer als die charakteristische Siebgröße sind, zerkleinert werden. Das Passieren erfolgt bevorzugt in Apparaten wie einer Siebmühle der Firma Frewitt oder der Firma Hosokawa Bepex GmbH. Partikel, die größer als die charakteristische Größe des Passiersiebs sind, können beim Einsatz der erfindungsgemäßen Trägermaterialien im Bereich der Suspensionskatalyse zur unerwünschten Sedimentationen der Absorbate führen und haben lange Diffusions bzw. Reaktionszeiten zur Folge. Weiterhin ist es vorteilhaft, wenn alle Siebfraktionen kleiner 400 μm abgetrennt werden. Diese kleinen Partikel wirken sich wie zuvor beschrieben negativ auf den Strömungswiderstand der Partikel und führen zu Druckverlusten in Festbettreaktoren.

Die Siebung kann mittels aller bekannten Techniken erfolgen, vorzugsweise mittels eines Schwingsiebs von Firmen wie Vibra, Engelsmann oder Allgeier. Es können mehrere Siebe oder mehrere Siebungsschritte durchgeführt werden.

Im zweiten Falle des erfindungsgemäßen Verfahrens, in dem die Kompaktierung der Kieselsäure vorzugsweise in einem Trockenkompaktor mit nachgeschaltetem Siebgranulator und Sieb durchgeführt werden, d.h. zunächst wir die vorgelegte Kieselsäure z.B. in einem Apparat der Firma Hosokawa Bepex GmbH wie Bepex L200/50 oder der Firma Alexanderwerk AG kompaktiert und danach wird das kompaktierte Material auf die gewünschte Kornfraktion fraktioniert.

In Schritt ii) des zweiten erfindungsgemäßen Verfahrens wird die trockene Ausgangskieselsäure kompaktiert, d.h. zu Stülpen verpresst, welche eine für die erfindungsgemäße Anwendung optimierte Partikelgröße und Härte aufweist. Die Härte kann dabei durch den Druck mit dem die Ausgangskieselsäuren kompaktiert werden gesteuert werden. Die Kompaktierung erfolgt bevorzugt bei einem spezifischen Anpressdruck von 0,5 bis 15 kN/cm Walzenbreite, besonders bevorzugt von 3 bis 12 kN/cm Walzenbreite und ganz besonders bevorzugt von 6 bis 10 kN/cm Walzenbreite und bei einer Temperatur von 10°C bis 90°C, besonders bevorzugt von 10°C bis 70°C. Weiterhin kann während der Kompaktierung eine Flüssigkeit, vorzugsweise Wasser, wässrige Lösungen wie Celluloselösungen oder Öle zugegeben werden, die geeignet sind als Binder zwischen den Partikeln zu fungieren. Die Flüssigkeit wird vorzugsweise in Anteilen von 1 bis 30 Gew.%, besonders bevorzugt in Anteilen von 1 bis 20 Gew.% und ganz besonders bevorzugt in Anteilen von 3 bis 15 Gew.% zugegeben. Des Weiteren kann während der Kompaktierung ein Feststoff, der geeignet ist als Binder zwischen den Partikeln zu fungieren, wie beispielsweise Cellulose, Wachse oder Polymere oder Monomere welche anschließend polymerisiert werden zugegeben werden. Der Feststoff wird in Anteilen von 0,1 bis 50 Gew.%, bevorzugt in Anteilen von 0,5 bis 15 Gew.%, besonders bevorzugt in Anteilen zwischen 0,5 und 8 Gew.% zugegeben.

Diese Trockenkompaktierung erfolgt bevorzugt derart, dass die trockene Ausgangskieselsäure in einer Kompaktierungseinheit zwischen zwei rotierenden Walzen verpresst wird, wobei besonders bevorzugt zumindest eine Walze Vertiefungen wie Riffeln, Mulden oder Kissen aufweist, deren charakteristische Abmessungen größer sind als die der zu erzeugenden Partikel. Die Walzen sind gerade oder konkav ausgeführt. Eine weiter besonders bevorzugte Ausführung besteht darin zumindest eine gelochte Zahnradwalze zu verwenden. Weiterhin kann es vorteilhaft sein, wenn zumindest eine Walze derart ausgestaltet ist, dass an der Walzenoberfläche ein Unterdruck erzeugt werden kann, durch den die zu kompaktierende Kieselsäure an die Walze angesaugt wird. Die Zufuhr der Kieselsäure zu der Kompaktierungseinheit kann mittels aller dem Fachmann bekannten Fördermittel wie Beispielsweise Förderschnecken, Doppelschnecken etc. erfolgen.

Nach der Kompaktierung werden die erhaltenen Stülpen durch ein Sieb mit einer charakteristischen Größe von 3000 µm passiert, wobei die Partikel, die größer als die charakteristische Siebgröße sind, zerkleinert werden. Das Passieren erfolgt bevorzugt in Apparaten wie einer Siebmühle der Firma Frewitt oder der Firma Hosokawa Bepex GmbH. Die Partikel, die größer als die charakteristische Größe des Passiersiebs sind, können beim Einsatz der erfindungsgemäßen Trägermaterialien im Bereich der Suspensionskatalyse zur unerwünschten Sedimentationen der Absorbate führen und haben lange Diffusions bzw. Reaktionszeiten zur Folge. Weiterhin wird die Siebfraktion kleiner 400 µm abgetrennt. Diese kleinen Partikel wirken sich wie zuvor beschrieben negativ auf den Strömungswiderstand einer Partikelschüttung aus und führen zu Druckverlusten in Festbettreaktoren.

Die mögliche Wasserdampfbehandlung an den fertigen getrockneten Granulaten kann in allen dafür geeigneten Apparaten geschehen, dies sind z.B. Bandtrockner, Drehrohrtrockner, Trockenschränke, Wirbelschichttrockner, u.s.w. Die Granulate werden einer Temperatur von 70°C - 400°C, bevorzugt 80°C - 300°C, besonders bevorzugt 90°C - 200°C und ganz besonders bevorzugt 106°C - 180°C ausgesetzt. Die Verweilzeit bei dieser Temperatur beträgt bis zu 16h, bevorzugt bis zu 12h, besonders bevorzugt bis zu 8h, ganz besonders bevorzugt bis zu 4h.

Die mögliche Calcinierung der Partikel kann in verschieden Apparaten wie z.B. Calcinieröfen, Band- oder Drehrohrcalcinierer, in Flash- oder Wirbelschichtcalcinierern erfolgen. Die Granulate werden dabei Temperaturen von 700°C - 1200°C ausgesetzt, bevorzugt 800°C - 1200°C, besonders bevorzugt 800°C - 1100°C. Die Verweilzeit hängt von der Calciniertemperatur und der gewünschten Partikelhärte ab. Die Verweilzeit in dem Prozess beträgt 1h, bevorzugt 20 min, besonders bevorzugt weniger als 10 min.

Die erfindungsgemäßen granulären Kieselsäuren können zur Herstellung von Absorbaten eingesetzt werden, wobei es sich bei den absorbierten Substanzen bevorzugt um Härtungsmittel oder Initiatoren, Vernetzungsmittel, Katalysatoren, pharmazeutische Wirk- und Hilfsstoffe, kosmetische Wirk-und Hilfsstoffe, Reinigungs- und/oder Pflegemittel, Geschmacks-, Aroma- und Duftstoffe, Futtermittel bzw. Futtermittelzusatzstoffe wie z.B. Aminosäuren, Vitamine, Mineralstoffe, Lebensmittel bzw. Lebensmittelzusatzstoffe, Farbstoffe und/oder Pigmente, Aminosäuren, Oxidations- oder Bleichmittel, Additive mit mikrobizider, insbesondere fungizider oder bakterizider Wirkung, Chemikalien für die Land- und Forstwirtschaft und/oder ein Betonzusatzstoffe handelt. Dabei kann es sich bei dem auf dem Träger absorbierten Material um eine wässrige oder nicht-wässrige Flüssigkeit, z.B. ein Öl, ein Harz, eine Lösung, eine Dispersion, eine Suspension, eine Emulsion, ein Wachs, ein Polymer oder eine Schmelze handeln. Die absorbierten Stoffe können anschließend thermisch behandelt, getempert, zur Kristallisation, zur Erstarrung, zur Entmischung oder zur Reaktion gebracht werden. Zusätzlich können die absorbierten Stoffe vor oder nachher getrocknet werden.

Absorbate im Bereich Futtermittel und Futtermittelzusatzstoffe umfassen z.B. Vitamine, Mineralstoffe, Carbonsäuren, Mineralsäuren, Aminosäuren Fette, Öle und Aromen. Besonders bevorzugt handelt es sich dabei um Ameisensäure, Essigsäure, Propionsäure, Milchsäure, Phosphorsäure, Cholinchloridlösung, Vitamin E-Acetat und Pflanzenextrakte wie zum Beispiel Tagetesextrakt.

Absorbate im Bereich Land- und Forstwirtschaft umfassen z.B. absorbierte Düngemittel wie z.B. nitrat - und/oder phosphathaltige Dünger, Pflanzenschutzmittel, Schädlingsbekämpfungsmittel wie z B. Herbizide, Fungizide, Insektizide.

Absorbate im Bereich kosmetische Erzeugnisse umfassen z.B. Öle wie etherischen Öle, Parfümöle, Pflegeöle, Duftöle und Silikonöle, antibakterielle, antivirale oder fungizide Wirkstoffe; desinfizierend und antimikrobiell wirkende Substanze; Desodorantien; Antioxidantien; biologisch wirksame Stoffe und biogene Wirkstoffe; Vitamine und Vitaminkomplexe; Enzyme und enzymatische Systeme wie Amylasen, Cellulasen, Lipasen und Proteasen; kosmetisch aktiven Substanze wie Inhaltsstoffe für Kosmetika und Körperpflegemittel; wasch- und reinigungsaktive Substanzen wie Tenside aller Art, wasch- und/oder reinigungsaktive anorganische und organische Säuren, Soil-repellent-und-Soil-release-Wirkstoffe, Oxidantien und Bleichmittel, Bleichmittelaktivatoren, Buildern und Cobuildern, Antiredepositionsadditive, Vergrauungs- und Verfärbungsinhibitoren, Wirksubstanzen zum Farbschutz, Substanzen und Additiven zur Wäschepflege, optischen Aufheller, Schauminhibitoren, pH-Stellmitteln und pH-Puffersubstanzen.

Absorbate im Bereich Lebensmittel bzw. Lebensmittelzusatzstoffe umfassen z.B. absorbierte Aromen, Nahrungsergänzungsmittel, Vitamine, Mineralstoffe, Aminosäuren.

Absorbate aus pharmazeutischen Wirkstoffen umfassen alle Arten von pharmazeutischen Wirkstoffen wie zum Beispiel $\alpha$-Proteinase-Inhibitor, Abacavir, Abciximab, Acarbose, Acetylsalicylsäure, Acyclovir, Adenosin, Albuterol, Aldesleukin, Alendronat, Alfuzosin, Alosetron, Alprazolam, Alteplase, Ambroxol, Amifostin, Amiodaron, Amisulprid, Amlodipin, Amoxicillin, Amphetamin, Amphotericin, Ampicillin, Amprenavir, Anagrelid, Anastrozol, Ancrod, Anti-Hämophiliefaktor, Aprotinin, Atenolol, Atorvastatin, Atropin, Azelastin, Azithromycin, Azulen, Barnidipin, Beclomethason, Benazepril, Benserazid, Beraprost, Betamethason, Betaxolol, Bezafibrat, Bicalutamid, Bisabolol, Bisoprolol, Botulinus-Toxin, Brimonidin, Bromazepam, Bromocriptin, Budesonid, Bupivacain, Bupropion, Buspiron, Butorphanol, Cabergolin, Calcipotrien, Calcitonin, Calcitriol, Campher, Candesartan, Candesartan cilexetil, Captopril, Carbamazepin, Carbidopa, Carboplatin, Carvedilol, Cefaclor, Cefadroxil, Cefaxitin, Cefazolin, Cefdinir, Cefepim, Cefixim, Cefmetazol, Cefoperazon, Cefotiam, Cefoxopran, Cefpodoxim, Cefprozil, Ceftazidim, Ceftibuten, Ceftriaxon, Cefuroxim, Celecoxib, Celiprolol, Cephalexin, Cerivastatin, Cetirizin, Chloramophenicol, Cilastatin, Cilazapril, Cimetidin, Ciprofibrat, Ciprofloxacin, Cisaprid, Cisplatin, Citalopram, Clarithromycin, Clavulansäure, Clindamycin, Clomipramin, Clonazepam, Clonidin, Clopidogrel, Clotrimazol, Clozapin, Cromolyn, Cyclophosphamid, Cyclosporin, Cyproteron, Dalteparin, Deferoxamin, Desogestrel, Dextroamphetamin, Diazepam, Diclofenac, Didanosin, Digitoxin, Digoxin, Dihydroergotamin, Diltiazem, Diphtherie-Protein, Diphtherie-Toxoxid, Divalproex, Dobutamin, Docetaxel, Dolasetron, Donepezil, Dornase-$\alpha$, Dorzolamid, Doxazosin, Doxifluridin, Doxorubicin, Dydrogesteron, Ecabet, Efavirenz, Enalapril, Enoxaparin, Eperison, Epinastin, Epirubicin, Eptifibatid, Erythropoietin-$\alpha$, Erythropoietin-$\beta$, Etanercept, Ethinylöstradiol, Etodolac, Etoposid, Faktor-VIII, Famciclovir, Famotidin, Faropenem, Felodipin, Fenofibrat, Fenoldopam, Fentanyl, Fexofenadin, Filgrastim, Finasterid, Flomoxef, Fluconazol, Fludarabin, Flunisolid, Flunitrazepam, Fluoxetin, Flutamid, Fluticason, Fluvastatin, Fluvoxamin, Follitropin-$\alpha$, Follitropin-$\beta$, Formoterol, Fosinopril, Furosemid, Gabapentin, Gadodiamid, Ganciclovir, Gatifloxacin, Gemcitabin, Gestoden, Glatiramer, Glibenclamid, Glimepirid, Glipizid, Glyburid, Goserelin, Granisetron, Griseofulvin, Hepatitis-B-Antigen, Hyaluronasäure, Hycosin, Hydrochlorthiazid, Hydrocodon, Hydrocortison, Hydromorphon, Hydroxychloroquin, Hylan G-F 20, Ibuprofen, Ifosfamid, Imidapril, Imiglucerase, Imipenem, Immunoglobulin, Indinavir, Indomethacin, Infliximab, Insulin, Insulin, human, Insulin Lispro, Insulin aspart, Interferon-$\beta$, Interferon-$\alpha$, Iod-125, Iodixanol, Iohexol, Iomeprol, Iopromid, Ioversol, Ioxoprolen, Ipratropium, Ipriflavon, Irbesartan, Irinotecan, Isosorbid, Isotretinoin, Isradipin, Itraconazol, Kaliumchlorazepat, Kaliumchlorid, Ketorolac, Ketotifen, Keuchhusten-Vakzin, Koagulationsfaktor-IX , Lamivudin, Lamotrigin, Lansoprazol, Latanoprost, Leflunomid, Lenograstim, Letrozol, Leuprolid, Levodopa, Levofloxacin, Levonorgestrel, Levothyroxin, Lidocain, Linezolid, Lisinopril, Lopamidol, Loracarbef, Loratadin, Lorazepam, Losartan, Lovastatin, Lysinacetylsalicylsäure, Manidipin, Mecobalamin, Medroxyprogesteron, Megestrol, Meloxicam, Menatetrenon, Meningokokken-Vakzin, Menotropin, Meropenem, Mesalamin, Metaxalon, Metformin, Methylphenidat, Methylprednisolon, Metoprolol, Midazolam, Milrinon, Minocyclin, Mirtazapin, Misoprostol, Mitoxantron, Moclobemid, Modafinil, Mometason, Montelukast, Morniflumat, Morphium, Moxifloxacin, Mykophenolat, Nabumeton, Nadroparin, Naproxen, Naratriptan, Nefazodon, Nelfinavir, Nevirapin, Niacin, Nicardipin, Nicergolin, Nifedipin, Nilutamid, Nilvadipin, Nimodipin, Nitroglycerin, Nizatidin, Norethindron, Norfloxacin, Octreotid, Olanzapin, Omeprazol, Ondansetron, Orlistat, Oseltamivir, Östradiol, Östrogene, Oxaliplatin, Oxaprozin, Oxolinsäure, Oxybutynin, Paclitaxel, Palivizumab, Pamidronat, Pancrelipase, Panipenem, Pantoprazol, Paracetamol, Paroxetin, Pentoxifyllin, Pergolid, Phenytoin, Pioglitazon, Piperacillin, Piroxicam, Pramipexol, Pravastatin, Prazosin, Probucol, Progesteron, Propafenon, Propofol, Propoxyphen, Prostaglandin, Quetiapin, Quinapril, Rabeprazol, Raloxifen, Ramipril, Ranitidin, Repaglinid, Reserpin, Ribavirin, Riluzol, Risperidon, Ritonavir, Rituximab, Rivastigmin, Rizatriptan, Rofecoxib, Ropinirol, Rosiglitazon, Salmeterol, Saquinavir, Sargramostim, Serrapeptase, Sertralin, Sevelamer, Sibutramin, Sildenafil, Simvastatin, Somatropin, Sotalol, Spironolacton, Stavudin, Sulbactam, Sulfaethidol, Sulfamethoxazol, Sulfasalazin, Sulpirid, Sumatriptan, Tacrolimus, Tamoxifen, Tamsulosin, Tazobactam, Teicoplanin, Temocapril, Temozolomid, Tenecteplase, Tenoxicam, Teprenon, Terazosin, Terbinafin, Terbutalin, Tetanus Toxoid , Tetrabenazin, Tetrazapam, Thymol, Tiagabin, Tibolon, Ticarcillin, Ticlopidin, Timolol, Tirofiban, Tizanidin, Tobramycin, Tocopherylnicotinat, Tolterodin, Topiramat, Topotecan, Torasemid, Tramadol, Trandolapril, Trastuzumab, Triamcinolon, Triazolam, Trimebutin, Trimethoprim, Troglitazon, Tropisetron, Tulobuterol, Unoproston, Urofollitropin, Valacyclovir, Valproinsäure, Valsartan, Vancomycin, Venlafaxin, Verapamil, Verteporfin, Vigabatrin, Vinorelbin, Vinpocetin, Voglibose, Warfarin, Zafirlukast, Zaleplon, Zanamivir, Zidovudin, Zolmitriptan, Zolpidem, Zopiclon und deren Derivate. Unter pharmazeutischen Wirkstoffen sind jedoch auch andere Substanzen wie Vitamine, Provitamine, essentielle Fettsäuren, Extrakte pflanzlicher und tierischer Herkunft, Öle pflanzlicher und tierischer Herkunft, pflanzliche Arzneizubereitungen und homöopathische Zubereitungen zu verstehen.

Die erfindungsgemäßen granulären Kieselsäuren können insbesondere als Träger für Futtermitteladditive wie z.B. Ameisensäure, Propionsäure, Milchsäure, Phosphorsäure, Cholinchloridlösung, Vitamin E-Acetat oder Pflanzenextrakten zum Beispiel Tagetesextrakt verwendet werden.

Weiterhin können die erfindungsgemäßen granulären Kieselsäuren als Trägermaterial für chemische Produkte wie Melaminharze, Gummiadditive, Kunststoffadditive, Additive für Bauchemikalien oder Lackadditive verwendet werden.

Ganz besonders bevorzugt werden die erfindungsgemäßen granulären Kieselsäuren als Trägermaterial für Katalysatoren aller Art eingesetzt. Bei den Katalysatoren kann es sich insbesondere bevorzugt um Enzyme oder eine Kombination von verschiedenen Enzymen wie z.B. Enzyme aus der Klasse der Oxidoreductasen, Transferasen, Hydrolasen, Lipasen, Lysasen, Isomerasen und Ligasen (gemäß EC (Enzyme Commision) Nummer des Nomenclature Committee of the International Union of Biochemistry and Molecular Biology) handeln. Enzymvarianten, die z.B. durch Rekombinationstechniken hergestellt wurden, sollen ebenfalls in dem Begriff Enzym eingeschlossen sein.

Zur Herstellung der beladenen Träger werden die erfindungsgemäßen granulären Kieselsäuren mit mindestens einer zu absorbierenden Substanz in Kontakt gebracht, so dass die Substanz in die Poren der Kieselsäure eindringen kann. Hierzu sind alle dem Fachmann bekannten Technologien wie z.B. Aufsprühen, Auftropfen, Tränken, Imprägnieren, Verdüsen etc. anwendbar. Bevorzugt wird die Kieselsäure in einer Feststoffmischeinheit wie z.B. Kneter, Schaufeltrockner, Taumelmischer, Vertikalmischer, Schaufelmischer, Schugimischer, Zementmischer, Gerickekontimischer, Eirichmischer und/oder Silomischer vorgelegt. Die Temperatur in der Mischeinheit beträgt in Abhängigkeit von der Art und Zusammensetzung der zu absorbierenden Substanz bevorzugt zwischen 5°C und 90°C, besonders bevorzugt zwischen 10°C und 70°C. Der Druck im Mischer beträgt bevorzugt zwischen 0,1 bar und 2 bar, besonders bevorzugt zwischen 0,5 bar und 1,2 bar.

Der Gehalt an absorbierter Substanz in den beladenen Trägern liegt zwischen 5 und 70 Gew.%, bevorzugt zwischen 5 und 65 Gew.%, besonders bevorzugt zwischen 5 und 60 Gew.%. Der Term absorbierte Substanz beschreibt die Summe aller auf den Träger aufgebrachten Stoffe.

Die erfindungsgemäßen Absorbate werden insbesondere bevorzugt als Katalysatoren in Festbettreaktoren, im Bereich der heterogenen Katalyse, in Wirbelschichtreaktoren und zur Reaktion in Suspensionen eingesetzt.

Die physikalisch/chemischen Daten der eingesetzten Rohstoffe und der erfindungsgemäßen granulären Kieselsäuren werden mit den folgenden Methoden bestimmt:

### Bestimmung der BET-Oberfläche

Die spezifische Stickstoff-Oberfläche (im Folgenden BET-Oberfläche genannt) Kieselsäure wird gemäß ISO 9277 als Multipoint-Oberfläche bestimmt. Als Messgerät dient das Oberflächenmessgerät TriStar 3000 der Firma Micromeritics. Die BET-Oberfläche wird üblicherweise in einem Partialdruckbereich von 0,05 - 0,20 des Sättigungsdampfdruckes des flüssigen Stickstoffs bestimmt. Die Probenvorbereitung erfolgt durch Temperierung der Probe für eine Stunde bei 160°C unter Vakuum in der Ausheizstation VacPrep 061 der Firma Micromeritics.

### Bestimmung der DBP-Aufnahme

Die DBP-Aufnahme (DBP-Zahl), die ein Maß für die Saugfähigkeit der Kieselsäure darstellt, wird in Anlehnung an die Norm DIN 53601 wie folgt bestimmt.

12,50 g Kieselsäure mit 3 - 10% Feuchtegehalt (gegebenenfalls wird der Feuchtegehalt durch Trocknen bei 105°C im Trockenschrank eingestellt) werden in die Kneterkammer des Absorptometers C der Firma Brabender gegeben. Die Messung am Absorptometer C erfolgt PC-gestützt unter Verwendung der Software BRABENDER Automatic Öl Absorption System Version 1.1.2 mit fest vorgegebener Dämpfung der Drehmomentmesskurve.

Im Falle von Filterkuchen wird dieser vor der Verwendung bei 105°C im Trockenschrank bis zu einem Feuchtegehalt von ≤ 10% getrocknet und über ein 3 mm Sieb sowie anschließend über ein 300 μm Sieb passiert.

Bei einer Umlaufgeschwindigkeit der linken Kneterschaufel von 125 U/min wird bei Raumtemperatur mithilfe der zum Absorptometer C gehörigen Bürette Titronic Universal (Firma Schott) Dibutylphthalat mit einer Geschwindigkeit von 4 ml/min in die Kneterkammer getropft. Der Abschaltpunkt, bei dem die Steuersoftware des Absorptometers C Kneter und DBP-Dosierung stoppt, wird bei einem Drehmoment von 0,6 Nm definiert.

Folgende Formel dient zur Berechnung der DBP-Aufnahme [g/100g]:

$$DBP = \frac{V * D * 100}{E} * \frac{g}{100\,g} + K$$

mit

DBP:   DBP-Aufnahme [g/100g]
V:       Verbrauch DBP [ml]
D:       Dichte DBP [g/ml] (1,047 g/ml bei 20°C)
E:       Einwaage Kieselsäure [g]
K:       Korrekturwert gemäß Feuchtekorrekturtabelle [g/100 g]

Die DBP-Aufnahme ist für wasserfreie, getrocknete Kieselsäuren definiert. Bei Verwendung von ungetrockneten Kieselsäuren ist der Korrekturwert K für die Berechnung der DBP-Aufnahme zu berücksichtigen. Dieser Wert kann anhand der folgenden Korrekturtabelle ermittelt werden.

**Tabelle 1:** Feuchtekorrekturtabelle für Dibutylphthalat-Aufnahme (wasserfrei)

| % Feuchte | ,% Feuchte | | | | |
|---|---|---|---|---|---|
| | ,0 | ,2 | ,4 | ,6 | ,8 |
| 0 | 0 | 2 | 4 | 5 | 7 |
| 1 | 9 | 10 | 12 | 13 | 15 |
| 2 | 16 | 18 | 19 | 20 | 22 |
| 3 | 23 | 24 | 26 | 27 | 28 |
| 4 | 28 | 29 | 29 | 30 | 31 |
| 5 | 31 | 32 | 32 | 33 | 33 |
| 6 | 34 | 34 | 35 | 35 | 36 |
| 7 | 36 | 37 | 38 | 38 | 39 |
| 8 | 39 | 40 | 40 | 41 | 41 |
| 9 | 42 | 43 | 43 | 44 | 44 |
| 10 | 45 | 45 | 46 | 46 | 47 |

Beispiel:

Beträgt der Feuchtegehalt einer Kieselsäure 5,8%, wird zu dem wie oben beschriebenen analysierten Wert für die DBP-Aufnahme ein Korrekturwert K von 33 g/100g addiert. Die Feuchte einer Kieselsäure wird gemäß der im weiteren Textverlauf beschriebenen Methode "Bestimmung der Feuchte" ermittelt.

**Bestimmung der Partikelgröße mittels Laserbeugung**

Die Anwendung der Laserbeugung zur Bestimmung von Partikelgrößenverteilungen pulverförmiger Feststoffe basiert auf dem Phänomen, dass Partikel in Abhängigkeit von ihrer Größe das Licht eines monochromen Laserstrahls mit differierenden Intensitätsmustern in alle Richtungen streuen bzw. beugen. Je kleiner der Durchmesser des angestrahlten Partikels ist, desto größer sind die Streuungs- bzw. Beugungswinkel des monochromen Laserstrahls.

**Probenvorbereitung für die Partikelgrößenmessung mittels Laserbeugung**

Da die Größe der Probenpartikel zum Teil den Messbereich des verwendeten Gerätes überschreitet und das Verhältnis von d50-Wert ohne Ultraschalleinwirkung zu d50U-Wert nach 3 min Ultraschalleinwirkung von der Ausgangspartikelgröße abhängt (kleinere Partikel eines Materials besitzen ein höheres Verhältnis der beschriebenen Größen), wird vor der Messung eine Partikelfraktion von 400 $\mu$m - 500 $\mu$m aus der Probe ausgesiebt. Durch dieses Vorgehen kann die Stabilität von verschiedenen Materialien zuverlässig verglichen werden und man erhält eine Aussage über die stoffspezifische Stabilität. Die Siebung erfolgt mit eine Siebmaschine HAVER EML 200 Digital Plus, Fa. Haver & Boecker, 59302 Oelde, das mit einem 400 $\mu$m und 500 $\mu$m Sieb ausgerüstet ist. Es werden 5 g des Ausgangsmaterials auf das obere 500 $\mu$m Sieb aufgegeben und 2 Minuten mit einer Amplitudeneinstellung von 1,0 gesiebt. Die Partikelfraktion zwischen 400 $\mu$m und 500 $\mu$m wird für die weitere Messung verwendet.

Sollte die für den Vergleich wichtige Fraktion 400 $\mu$m bis 500 $\mu$m nicht Bestandteil der Partikelgrößenverteilung des vorliegenden Trägermaterials sein, wird eine entsprechende Siebfraktion hergestellt, indem eine ausreichende Menge des Ausgangsmaterials mit Hilfe eines Siebgranulators der Firma Eweka GmbH, Heusenstamm, Typ TG2S bei 100 Oszillationen / Minute über ein 500 $\mu$m Sieb passiert und anschließend über ein 400 $\mu$m Sieb abgesiebt wird. Die Siebung geschieht wie oben beschrieben.

**Bestimmung d$_{50}$-Wert ohne Ultraschalleinwirkung**

Die Probenvorbereitung für die Messung (Spülen des Moduls usw.) mittels Laserbeugungsgerät LS 230 (Fa. Beckman Coulter; Messbereich 0,04 - 2000 $\mu$m) und Flüssigkeitsmodul (Small Volume Module Plus, 120 ml, Fa. Beckman Coulter mit integriertem Ultraschall-Finger) erfolgt im Falle hydrophiler Kieselsäuren mithilfe von 0,05% m/m Tetra-Natriumdiphosphat in VE-Wasser als Dispergierflüssigkeit, im Falle nicht ausreichend mit Wasser benetzbarer Kieselsäuren mit einem Ethanol/Wassergemisch (Volumenverhältnis 1:1) als Dispergierflüssigkeit. Vor Beginn der Messung muss das Laserbeugungsgerät 2 Stunden warmlaufen. Danach wird das SVM-Modul dreimal mit der Dispergierflüssigkeit gespült. Folgende für die Partikelmessung relevante Parameter sind einzustellen:

| Messzeit: | 60 Sekunden |
|---|---|
| Anzahl der Messungen: | 1 |
| Pumpengeschwindigkeit: | 75% |
| Optisches Modell: | Fraunhofer |
| PIDS-Funktion: | deaktiviert |
| Offsetmessung: | aktiviert |
| Justierung: | Auto |
| Hintergrundmessung: | aktiviert |
| Probenkonzentration einstellen: | aktiviert |

Mittels Spatel erfolgt die Zugabe der Kieselsäure-Siebfraktion (400 - 500 $\mu$m) bis zum Erreichen der erforderlichen Messkonzentration, die der Laserbeuger LS 230 mit "OK" meldet. Nach Dispergierung der Kieselsäuresuspension für 60 Sekunden durch Umpumpen ohne Ultrabeschallung erfolgt die Messung bei Raumtemperatur. Aus der Rohdatenkurve berechnet die Software auf Basis des Fraunhofer Modells (Fraunhofer.rfd-Datei) die Partikelgrößenverteilung und den $d_{50}$-Wert ohne Ultraschalleinwirkung (Medianwert).

## Bestimmung $d_{50U}$-Wert nach 3 Minuten Ultrabeschallung bei 100% Amplitude

Die im Laserbeuger LS 230 befindliche KieselsäureSuspension wird durch Ultrabeschallung für 180 Sekunden mittels im SVM-Modul integriertem Ultraschall-Finger (Ultraschallprozessor Vibra Cell VCX 130 der Firma Sonics mit Ultraschallkonverter CV 181 und 6 mm Ultraschallspitze) bei 100% Amplitude und gleichzeitigem Umpumpen im Flüssigkeitsmodul erneut dispergiert und wie oben beschrieben gemessen.

Aus der Rohdatenkurve berechnet die Software auf Basis des Fraunhofer Modells (Fraunhofer.rfd-Datei) die Partikelgrößenverteilung und den $d_{50U}$-Wert nach 3 Minuten Ultraschallwirkung (Medianwert).

## Bestimmung der Partikelgröße mittels dynamischer Bildauswertung

Bei der dynamischen Bildauswertung fällt ein Schüttgutstrom zwischen einer Lichtquelle und einer Kamera herab. Die Partikel werden als Projektionsfläche erfasst, digitalisiert und mit einem Computerprogramm in eine Partikelgröße umgerechnet.

## Bestimmung des dQ3=10%-Wert und des dQ3=90%-Wert

Zur Messung der Partikelgröße wird der CAMSIZER der Firma RETSCH Technology GmbH, Haan eingesetzt. Die Partikel werden mit Hilfe der Dosierrinne DR 100-40 mit Vorratstrichter dem Messgerät zugeführt. Für die Bildauswertung ist die mitgelieferte Software in der Version 3.12d zu verwenden.

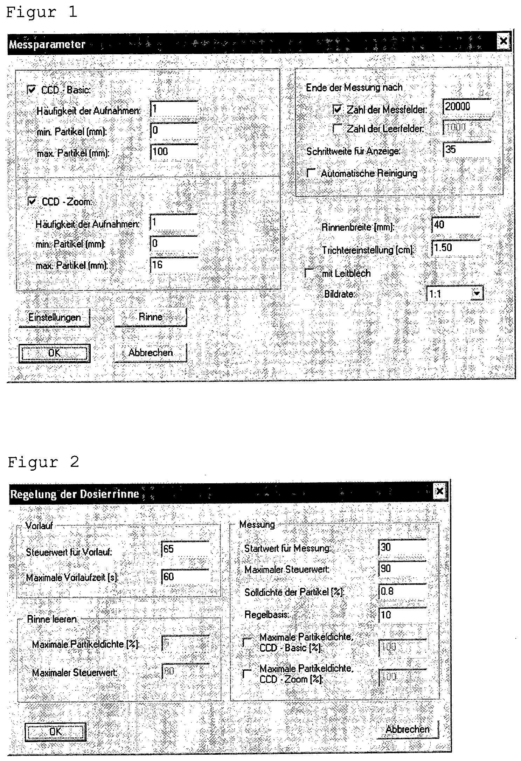

Vor Beginn der Messung lässt man das Gerät 2h warmlaufen. Es wird sichergestellt, dass Schutzgläser vor der Beleuchtungseinheit und der Kamera staubfrei sind. Der Abstand zwischen Trichter und Dosierrinne wird ca. auf das dreifache der maximalen Partikelgröße eingestellt. Die Dosierrinne wird direkt über dem Messgerät platziert. Es wird ca. 150 mL Probe in den Trichter eingefüllt. In der Meßaufgabendatei (*.afg) werden die Parameter für die Messung gemäß Figur 1 hinterlegt.

Zur Regelung der Dosierrinne werden die Einstellungen in der Software gemäß Figur 2 hinterlegt.

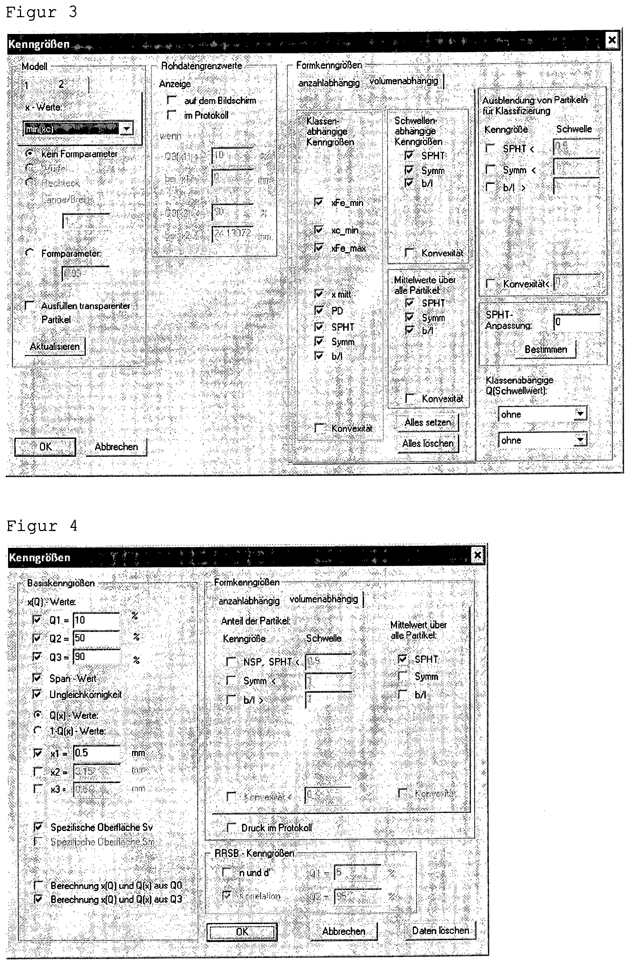

Bei der Auswertung der digitalisierten Bilder werden die x-Werte aus den min(xc) Werten berechnet. Es werden gemäß Figur 3 keine Formfaktoren verwendet.

Die Ausgabe des $d_{Q3-10\%}$-Wert und des $d_{Q3=90\%}$-Wert wird bei den Basiskenngrößen festgelegt (Figur 4).

Es wird keine Meßdatenanpassung mit Hilfe von sogenannten Anpassungsdateien vorgenommen.

## Bestimmung der Feuchte

Die Feuchte von Kieselsäuren wird gemäß ISO 787-2 bestimmt. Hierzu wird eine Probenmenge von 1 - 4 g in einem Trockenschrank bei (105 $\pm$ 2)°C für 2 Stunden getrocknet und entsprechend den ISO-Vorgaben ausgewertet. Dieser Trocknungsverlust besteht überwiegend aus physikalisch gebundenem Wasser.

**Bestimmung des pH-Wertes der Kieselsäure**

Die Bestimmung des pH-Wertes der Kieselsäure erfolgt als wässrige Suspension bei Raumtemperatur. Granulierte Proben werden vorab gemörsert oder vermahlen. Es werden zu 5 g Kieselsäure 95 g entionisiertes Wasser gegeben. Die Suspension wird mittels Magnetrührer 5 Minuten gerührt. Direkt im Anschluss wird mithilfe eines im zu erwartenden Messbereich kalibrierten pH-Meters (Metrohm 780 pH Meter) der pH-Wert der Suspension auf eine Dezimalstelle genau gemessen.

**Bestimmung des Quecksilber-Porenvolumens $\leq 4\,\mu$m**

Die Methode basiert auf der Quecksilber-Intrusion gemäß DIN 66133, wobei ein AutoPore IV 9520-Gerät der Firma Micromeritics verwendet wird.

Das Verfahrenprinzip beruht auf der Messung des in einen porösen Feststoff eingepressten Quecksilbervolumens in Abhängigkeit von dem angewendeten Druck. Dabei werden nur die Poren erfasst, in die bei dem angewendeten Druck (max. 414 MPa) Quecksilber eindringen kann (Verfahren von Ritter und Drake).

Eine nichtbenetzende Flüssigkeit dringt nur unter Druck in ein poröses System ein. Der aufzuwendende Druck ist umgekehrt proportional zur lichten Weite der Porenöffnungen. Für zylindrische Poren ist der Zusammenhang zwischen Porenradius $r_p$ und Druck p durch die Washburn-Gleichung gegeben:

$$r_p = -\frac{2 \times \sigma}{p} \times \cos\theta$$

$r_p$ : Porenradius
p : Druck
$\sigma$ : Oberflächenspannung (480 mN/m*)
$\theta$ : Kontaktwinkel des Quecksilbers (140°*) * gemäß DIN 66133

Das Quecksilber-Porenvolumen $\leq 4\,\mu$m ergibt sich aus dem kumulierten Porenvolumen aller Poren mit einem Durchmesser von $\leq 4\,\mu$m bis zur Bestimmungsgrenze des Quecksilber-Porosimeters AutoPore IV 9520 (Maximaldruck 414 MPa).

Die folgenden Beispiele sollen die Erfindung näher erläutern, ohne ihren Umfang zu beschränken.

Beispiel 1

SIPERNAT®50S der Firma Evonik Degussa GmbH wurde in einem Mischer (Firma Somakon, Typ MP-L1) unter Zusatz von 200 ml Wasser / 100 g Silica gemischt und verdichtet. Dabei wurde der auf 23°C temperierte 0,5 Liter Mischbehälter, ausgestattet mit dem Standardmischkreuz verwendet. Zu den eingewogenen 15 g Silica werden bei einer Mischgeschwindigkeit von 2200 Upm innerhalb von 20 Sekunden 30 g Wasser dosiert und anschließend solange gemischt bis es zur Granulation kommt. Der Prozess wird angehalten sobald äußerlich leicht feuchte 5 mm Agglomerate entstanden sind. Die erhaltenen Granulate werden bei 160°C im Trockenschrank bis zur Gewichtskonstanz getrocknet, anschließend über ein 500 $\mu$m Sieb passiert und in einem weiteren Arbeitsschritt über ein 400 $\mu$m Sieb gesiebt. Die so erhaltene Siebfraktion 400 - 500 $\mu$m wird für die anschließende Prüfung der Härte und der Porosität verwendet.

**Beispiel 2**

SIPERNAT®50S der Firma Evonik Degussa GmbH wurde in einem Mischer (Firma Somakon, Typ MP-L1) unter Zusatz von 270 ml Wasser / 100 g Silica gemischt und verdichtet. Dabei wurde der auf 23°C temperierte 0,5 Liter Mischbehälter, ausgestattet mit dem Standardmischkreuz verwendet. Zu den eingewogenen 15 g Silica werden bei einer Mischgeschwindigkeit von 2200 Upm innerhalb von 20 Sekunden 40,5 g Wasser dosiert und anschließend solange gemischt bis es zur Granulation kommt. Der Prozess wird angehalten sobald äußerlich leicht feuchte 5 mm Agglomerate entstanden sind. Die erhaltenen Granulate werden bei 160°C im Trockenschrank bis zur Gewichtskonstanz getrocknet, anschließend über ein 500 $\mu$m Sieb passiert und in einem weiteren Arbeitsschritt über ein 400 $\mu$m Sieb gesiebt. Die so erhaltene Siebfraktion 400 - 500 $\mu$m wird für die anschließende Prüfung der Härte und der Porosität verwendet.

**Beispiel 3**

Eine Probe der unter Beispiel 2 hergestellten Trägerkieselsäure wird für 16 Stunden bei 110°C unter Wasserdampfatmosphäre gelagert, anschließend bei 120°C bis zur Gewichtskonstanz nachgetrocknet und für die anschließende Prüfung der Härte und der Porosität verwendet.

### Beispiel 4

Filterkuchen einer SIPERNAT® 22 (Fa. Evonik Degussa GmbH) Suspension mit einem Feststoffgehalt von ca. 25% wird vorzerkleinert in einen Trommelgranulator (Fa. RWK) gegeben. Bei einem Füllgrad von 20%, einer Drehzahl von 8 U/min, einer Batchzeit von 90 Minuten sowie einer Heiztemperatur von 120°C entstehen trockene Granulate. Die Granulate werden anschließend in einem Siebgranulator (Fa. Frewitt, MG 633) mit einem Siebeinsatz von 1250 $\mu$m auf eine definierte maximale Partikelgröße gebrochen. Um ein staubfreies Produkt zu erhalten, wird der Feinanteil durch Siebung (Fa. Gough, Vibrecon GV 2/1, Ø 600 mm) über ein 400 $\mu$m Sieb abgetrennt. Dieser Feinanteil kann zusammen mit dem Filterkuchen bei dem nächsten Granulationsbatch mit eingesetzt werden. Die erhaltenen Granulate werden bei 160°C im Trockenschrank bis zur Gewichtskonstanz getrocknet, anschließend über ein 500 $\mu$m Sieb passiert und in einem weiteren Arbeitsschritt über ein 400 $\mu$m Sieb gesiebt. Die so erhaltene Siebfraktion 400 - 500 $\mu$m wird für die anschließende Prüfung der Härte und der Porosität verwendet.

### Beispiel 5

Filterkuchen einer SIPERNAT® 22 (Fa. Evonik Degussa GmbH) Suspension mit einem Feststoffgehalt von ca. 25% wird vorzerkleinert in einen Trommelgranulator (Fa. RWK) gegeben. Bei einem Füllgrad von 20%, einer Drehzahl von 8 U/min, einer Batchzeit von 90 Minuten sowie einer Heiztemperatur von 120°C entstehen trockene Granulate.
5,0 g der so erhaltenen Granulate werden in eine Porzellanschale (Masse: 154 g; Durchmesser: 120 mm) eingewogen und in einen auf 1000°C vorgeheizten Laborofen (Nabertherm) eingestellt. Nach 5 Minuten wird die Probe entnommen und sofort in ein kaltes Glasgefäß umgefüllt. Die erkalteten Granulate werden anschließend über ein 500 $\mu$m Sieb passiert und in einem weiteren Arbeitsschritt über ein 400 $\mu$m Sieb gesiebt. Die so erhaltene Siebfraktion 400 - 500 $\mu$m wird für die anschließende Prüfung der Härte und der Porosität verwendet.

### Beispiel 6

SIPERNAT® 22 der Firma Evonik Degussa GmbH wird mit einem Kompaktor (Fa. Bepex, L200/50) bei einer Anpresskraft der Walzen von 40 kN verpresst. Die Kompaktate werden anschließend in einem Siebgranulator (Fa. Frewitt, MG 633) mit einem Siebeinsatz von 2800 $\mu$m auf eine definierte maximale Partikelgröße gebrochen. Um ein staubfreies Produkt zu erhalten, wird der Feinanteil von den Granulation durch Siebung (Fa. Gough, Vibrecon GV 2/1, Ø 600 mm) über ein 400 $\mu$m Sieb abgetrennt und in die Vorlage des Kompaktors zurückgeführt. Für die Prüfung wird das Granulat über ein 500 $\mu$m Sieb gesiebt und in einem weiteren Arbeitsschritt über ein 400 $\mu$m Sieb gesiebt. Die so erhaltene Siebfraktion 400 - 500 $\mu$m wird für die anschließende Prüfung der Härte und der Porosität verwendet.

### Beispiel 7

SIPERNAT®50S der Firma Evonik Degussa GmbH wurde in einem Mischer (Firma Somakon, Typ MP-L1) unter Zusatz von 233 ml Wasser / 100 g Silica gemischt und verdichtet. Dabei wurde der auf 23°C temperierte 0,5 Liter Mischbehälter, ausgestattet mit dem Standardmischkreuz verwendet. Zu den eingewogenen 15 g Silica werden bei einer Mischgeschwindigkeit von 2200 Upm innerhalb von 20 Sekunden 35 g Wasser dosiert und anschließend solange gemischt es zur Granulation kommt. Der Prozess wird angehalten sobald äußerlich leicht feuchte 5 mm Agglomerate entstanden sind. Die erhaltenen Granulate werden bei 160°C im Trockenschrank bis zur Gewichtskonstanz getrocknet, anschließend über ein 500 $\mu$m Sieb passiert und in einem weiteren Arbeitsschritt über ein 400 $\mu$m Sieb gesiebt. Die so erhaltene Siebfraktion 400 - 500 $\mu$m wird für die anschließende Prüfung der Härte und der Porosität verwendet.
Die physikalisch chemischen Eigenschaften der erfindungsgemäßen Kieselsäuren nach Beispiel 1-7 werden in nachfolgender Tabelle 2 aufgeführt.

### Vergleichsbeispiele

Tabelle 2 enthält Angaben über die physikalisch-chemischen Eigenschaften von Vergleichskieselsäuren des Standes der Technik. Vergleichsbeispiel A und B entsprechen Ultrasil® 7000 GR und Ultrasil® VN3 GR der Firma Evonik Degussa GmbH. Bei Vergleichsbeispiel C handelt es sich um Zeosil® 165 GR® der Firma Rhodia Chimie. Bei Vergleichsbeispiel D handelt es sich um Zeodent DP-9175 der Firma Huber. Die Kieselsäuren der Vergleichsbeispiele A-C werden kommerziell zur Verstärkung von Kautschuk für Autoreifen eingesetzt.

**Tabelle 2:**

| Beispiel / Vergleichs-beispiel | Hg-Porenvolumen < 4µm [ml/g] | Verhältnis d50 durch d50U (Fraktion 400-500µm) | dQ3=10% [µm] | dQ3=90% [µm] |
|---|---|---|---|---|
| 1 | 1,20 | 1,42 | 467 | 994 |
| 2 | 1, 97 | 2,50 | 446 | 995 |
| 3 | 1,90 | 1,58 | 442 | 989 |
| 4 | 1,67 | 3, 03 | 562 | 923 |
| 5 | 0,95 | 1,09 | 428 | 977 |
| 6 | 1,05 | 1,47 | 563 | 2448 |
| 7 | 1,62 | 1,34 | 444 | 964 |
|  |  |  |  |  |
| A | 1,83 | 21,34 | 439 | 6292 |
| B | 1,63 | 5,40 | 955 | 5538 |
| C | 1,60 | 16, 67 | 316 | 5311 |
| D | 1,31 | 1,74 | 299 | 603 |

In Tabelle 2 zeigen die Beispiele 1 und 2 deutlich, dass, verursacht durch das Kompaktieren der Ausgangskieselsäure, die Partikel in ihrer Stabilität, gemessen durch das Verhältnis von $d_{50U}$-Wert ohne Ultraschalleinwirkung zu $d_{50}$-Wert nach 3 min Ultraschalleinwirkung, mit abnehmendem Wassergehalt beim Befeuchten zunehmen. Gleichzeitig verringert sich allerdings die Saugfähigkeit, ausgedrückt durch das Hg-Porenvolumen.

Durch die beschriebenen Herstellverfahren wird sichergestellt, dass die Produkte aus den Beispielen 1-7 nur einen sehr geringen Feinanteil aufweisen, ausgedrückt durch den dQ3=10% Wert von mehr als 400 µm.

Weiterhin zeigt Beispiel 3, dass die Nachbehandlung der erfindungsgemäßen Kieselsäuren einen überraschend deutlichen Gewinn an Partikelstabiltät bei nahezu unveränderter Porosität generiert.

Beispiel 5 zeigt, dass durch die Calcinierung Partikel mit extrem hoher Härte erzielt werden können.

Die in den Vergleichsbeispielen A bis C getesteten Trägerkieselsäuren weisen zwar eine verhältnismäßig hohe Saugfähigkeit auf, sind jedoch für Anwendungen in katalytischen Verfahren ungeeignet, da sie eine zu geringe Härte aufweisen (gekennzeichnet durch das Verhältnis $d_{50}$ durch $d_{50U}$). Zudem sind die $d_{Q3}$=90% Werte deutlich größer als 3000 µm wodurch in der Anwendung als Katalysatorträger die Diffusionswege für Edukte und Produkte in dem Porensystem der Kieselsäure lang sind

Die im Vergleichsbeispielen D getestete Trägerkieselsäure zeichnet sich durch eine ausreichende Saugfähigkeit und Härte aus, hat aber einen zu geringen $d_{Q3}$=10% Wert der in Reaktoren zu erhöhten Druckverlusten führt und den Strömungswiderstand der Absorbate erhöht.

Dies belegt, dass die erfindungsgemäßen granulären Kieselsäuren einen ausreichend geringen Feinanteil besitzen, sich bei gleichzeitig ausreichender Stabilität und Porosität damit deutlich von den bislang üblicherweise kommerziell eingesetzten Trägerkieselsäuren unterscheiden.

**Patentansprüche**

**1.** Granuläre Kieselsäure mit

- einem Hg-Porenvolumen (< 4 µm) von mehr als 0,90 ml/g,
- einem $d_{Q3=10\%}$-Wert von mehr als 400 µm bei gleichzeitig einem $d_{Q3=90\%}$-Wert von weniger als 3000 µm, wobei die Granulate durch Siebung oder Siebgranulation bei einer Siebgröße von 3000 µm und Absiebung des Feinanteils mit einer Siebmaschenweite von 400 µm auf die entsprechende Partikelfraktion eingestellt wird, und
- einem Verhältnis des $d_{50}$-Werts ohne Ultraschalleinwirkung zu $d_{50}$-Wert nach 3 min Ultraschalleinwirkung von < 4,00 aufweist, wobei die Messung an einer Fraktion von Partikeln von 400 bis 500 µm erfolgt.

**2.** Granuläre Kieselsäure nach Anspruch 1,
   **dadurch gekennzeichnet, dass** sie einen pH-Wert im Bereich von 5 bis 8,5 aufweist.

**3.** Granuläre Kieselsäure nach Anspruch 1 - 2,
   **dadurch gekennzeichnet, dass** sie ein Verhältnis des $d_{50}$-Werts ohne Ultraschalleinwirkung zu $d_{50}$-Wert nach 3 min Ultraschalleinwirkung von 1,00 bis 3,00, bevorzugt 1,00 bis 2,60, besonders bevorzugt 1,00 bis 2,10, speziell bevorzugt 1,00 bis 1,60 aufweist, die Messung erfolgt dabei an einer Fraktion von Partikeln von 400 bis 500 $\mu$m.

**4.** Granuläre Kieselsäure nach Anspruch 1 - 2,
   **dadurch gekennzeichnet, dass** sie ein Hg-Porenvolumen (<4$\mu$m) von mehr als 1,35 ml/g, bevorzugt mehr als 1,60 ml/g, besonders bevorzugt mehr als 1,80 ml/g, speziell bevorzugt mehr als 1,90 ml/g aufweist.

**5.** Granuläre Kieselsäure nach Anspruch 1 - 2,
   **dadurch gekennzeichnet, dass** sie ein Hg-Porenvolumen (<4$\mu$m) von 0,9 bis 1,34 ml/g, besonders bevorzugt von 0,9 ml/g bis 1,30 ml/g, besonders bevorzugt von 0,9 ml/g bis 1,20 ml/g aufweist.

**6.** Verfahren zur Herstellung von granulären Kieselsäuren umfassend die Schritte

   a) Bereitstellen einer gefällten oder pyrogenen Kieselsäure, getrocknet und/oder vermahlen mit

   - einer mittleren Partikelgröße $d_{50}$ ohne Ultraschallbehandlung von 0,1 bis 350 $\mu$m,
   - einer BET-Oberfläche von 30 bis 800 m²/g, und
   - einer DBP-Zahl von 140 bis 400 g/100g;

   b) Befeuchtung der Kieselsäure aus Schritt a) entsprechend des angewendeten Formgebungsverfahren auf einen Trocknungsverlust von 30-80 Gew.%;
   c) Formgebung der Kieselsäure aus Schritt b) durch Extrusion, Granulation, Kompaktierung, oder andere übliche Formgebungsverfahren;
   d) Trocknung der Kieselsäureformkörper in dafür geeigneten Trocknungsaggregaten; und
   e) Siebgranulation oder Siebung der Granulate bei einer Siebgröße von 3000 $\mu$m und Absiebung des Feinanteils mit einer Siebmaschenweite von 400 $\mu$m.

**7.** Verfahren nach Anspruch 6,
   **dadurch gekennzeichnet, dass** ein wasserhaltiger Filterkuchen mit einem Trocknungsverlust von 30-80 Gew.% als Ausgangsmaterial für Schritt c) verwendet wird.

**8.** Verfahren nach Anspruch 6,
   **dadurch gekennzeichnet, dass** die Kieselsäure aus Schritt a) in Schritt c) in schnell laufenden Intensivmischern verdichtet und granuliert wird.

**9.** Verfahren zur Herstellung von granulären Kieselsäuren umfassend die Schritte

   i) Bereitstellen einer gefällten oder pyrogenen Kieselsäure, getrocknet und/oder vermahlen mit einem Trocknungsverlust < 30 Gew.%, und mit

   - einer mittleren Partikelgröße $d_{50}$ ohne Ultraschallbehandlung von 0,1 bis 350 $\mu$m,
   - einer BET-Oberfläche von 30 bis 800 m²/g, und
   - einer DBP-Zahl von 140 bis 400 g/100g;

   ii) Formgebung der Kieselsäure aus Schritt i) durch Trockenkompaktierung, vorzugsweise zwischen zwei rotierenden Walzen, bei einem spezifischen Anpressdruck von 0,5 kN/cm Walzenbreite bis 12 kN/cm Walzenbreite zu Stülpen, und
   iii) Siebgranulation oder Siebung der Granulate bei einer Siebgröße von 3000 $\mu$m und Absiebung des Feinanteils mit einer Siebmaschenweite von 400 $\mu$m.

**10.** Verfahren nach einem der Ansprüche 6 bis 9,
   **dadurch gekennzeichnet, dass** alle Siebfraktionen kleiner 400 $\mu$m abgetrennt werden.

**11.** Verfahren nach einem der Ansprüche 6 oder 9,
**dadurch gekennzeichnet, dass** die Formgebungsschritte c) und ii) ohne die Zuführung von Bindern durchgeführt werden.

**12.** Verwendung der granulären Kieselsäuren nach einem der Ansprüche 1 bis 5 zur Herstellung von Absorbaten.

**13.** Absorbate umfassend zumindest eine der granulären Kieselsäuren nach einem der Ansprüche 1 bis 5.

**14.** Absorbate nach Anspruch 13,
**dadurch gekennzeichnet, dass** sie zumindest eine katalytisch aktive Substanz enthalten.

**15.** Absorbat nach einem der Ansprüche 13 oder 14,
**dadurch gekennzeichnet**, die absorbierten Substanzen in Anteilen von 1 bis 70 Gew.%, bevorzugt in Anteilen von 3 bis 60 Gew.% und besonders bevorzugt in Anteilen von 5 bis 50 Gew.% aufgebracht wird.

**16.** Absorbat nach einem der Ansprüche 13 oder 14,
**dadurch gekennzeichnet**, das der Aktivstoff oder Aktivstoffgemisch in Anteilen von 1 bis 20 Gew.%, bevorzugt in Anteilen von 3 bis 20 Gew.% und besonders bevorzugt in Anteilen von 5 bis 15 Gew.% aufgebracht wird.

**17.** Verfahren zur Herstellung von Absorbaten nach einem der Ansprüche 13 bis 16, **dadurch gekennzeichnet, dass** eine granuläre Kieselsäure nach einem der Ansprüchen 1 bis 5 mit zumindest einer Flüssigkeit ausgewählt aus der Gruppe bestehend aus Härtungsmittel oder Initiatoren, Vernetzungsmittel, Katalysatoren, pharmazeutische Wirk- und Hilfsstoffe, kosmetische Wirk- und Hilfsstoffe, Reinigungs- und/oder Pflegemittel, Geschmacks-, Aroma- und Duftstoffe, Futtermittel bzw. Futtermittelzusatzstoffen, Lebensmittel bzw. Lebensmittelzusatzstoffe, Farbstoffe und/oder Pigmente, Aminosäuren, Oxidations- oder Bleichmittel, Additive mit mikrobizider, insbesondere fungizider oder bakterizider Wirkung, Chemikalien für die Land- und Forstwirtschaft und/oder ein Betonzusatzstoff in Kontakt gebracht wird.

**18.** Verwendung der Absorbate nach einem der Ansprüche 13 bis 16 in katalytischen Verfahren.

**19.** Verfahren nach Anspruch 18,
**dadurch gekennzeichnet, dass** das Verfahren in einem Festbettreaktor, einem Wirbelschichtreaktor oder durch suspendieren der Absorbate in einer Reaktionsmischung durchgeführt wird.

**20.** Verwendung nach einem der Ansprüche 18 oder 19,
**dadurch gekennzeichnet, dass** die Absorbate ein Enzym als Katalysator auf einer granulären Kieselsäure nach einem der Ansprüche 1 bis 5 enthalten.

**Claims**

**1.** Granular silica having

- an Hg pore volume (< 4 $\mu$m) of more than 0.90 ml/g,
- a $d_{Q3=10\%}$ of more than 400 $\mu$m with, at the same time, a $d_{Q3=90\%}$ of less than 3000 $\mu$m, where the granules is adjusted to the appropriate particle fraction by screening or screen granulation at a screen size of 3000 $\mu$m and screening off the fines with a sieve mesh size of 400 $\mu$m, and
- a ratio of the $d_{50}$ without ultrasound exposure to $d_{50}$ after 3 min of ultrasound exposure of < 4.00, the measurement being effected on a fraction of particles from 400 to 500 $\mu$m.

**2.** Granular silica according to Claim 1,
**characterized in that** it has a pH in the range from 5 to 8.5.

**3.** Granular silica according to Claim 1-2,
**characterized in that** it has a ratio of the $d_{50}$ without ultrasound exposure to $d_{50}$ after 3 min of ultrasound exposure of 1.00 to 3.00, preferably 1.00 to 2.60, more preferably 1.00 to 2.10, especially preferably 1.00 to 1.60. This measurement is effected on a fraction of particles from 400 to 500 $\mu$m.

4. Granular silica according to Claim 1-2,
**characterized in that** it has an Hg pore volume (< 4 $\mu$m) of more than 1.35 ml/g, preferably more than 1.60 ml/g, more preferably more than 1.80 ml/g, especially preferably more than 1.90 ml/g.

5. Granular silica according to Claim 1-2,
**characterized in that** in has an Hg pore volume (< 4 $\mu$m) of 0.9 to 1.34 ml/g, more preferably of 0.9 ml/g to 1.30 ml/g, more preferably of 0.9 ml/g to 1.20 ml/g.

6. Process for producing granular silicas, comprising the steps of

    a) providing a precipitated or fumed silica, in dried and/or ground form, having

        - a mean particle size $d_{50}$ without ultrasound treatment of 0.1 to 350 $\mu$m,
        - a BET surface area of 30 to 800 m$^2$/g, and
        - a DBP number of 140 to 400 g/100 g;

    b) moistening the silica from step a) according to the shaping process employed to a drying loss of 30-80% by weight;
    c) shaping the silica from step b) by extrusion, granulation, compaction, or other customary shaping processes;
    d) drying the shaped silica bodies in drying units suitable therefor; and
    e) screen granulation or screening of the granules at a screen size of 3000 $\mu$m and screening off the fines with a screen mesh size of 400 $\mu$m.

7. Process according to Claim 6,
**characterized in that** a water-containing filtercake with a drying loss of 30-80% by weight is used as the starting material for step c).

8. Process according to Claim 6,
**characterized in that** the silica from step a) is, in step c), compacted and granulated in high-speed intensive mixers.

9. Process for producing granular silicas, comprising the steps of

    i) providing a precipitated or fumed silica, in dried and/or ground form, having a drying loss of < 30% by weight, and having

        - a mean particle size $d_{50}$ without ultrasound treatment of 0.1 to 350 $\mu$m,
        - a BET surface area of 30 to 800 m$^2$/g, and
        - a DBP number of 140 to 400 g/100 g;

    ii) shaping the silica from step i) by dry compaction, preferably between two rotating rollers, at a specific contact pressure of 0.5 kN/cm of roller width to 12 kN/cm of roller width to give slugs, and
    iii) screen granulation or screening of the granules at a screen size of 3000 $\mu$m and screening off the fines with a screen mesh size of 400 $\mu$m.

10. Process according to any of Claims 6 to 9,
**characterized in that** all screen fractions smaller than 400 $\mu$m are removed.

11. Process according to either of Claims 6 and 9,
**characterized in that** shaping steps c) and ii) are performed without the addition of binders.

12. Use of the granular silicas according to any of Claims 1 to 5 for production of absorbates.

13. Absorbates comprising at least one of the granular silicas according to any of Claims 1 to 5.

14. Absorbates according to Claim 13,
**characterized in that** they comprise at least one catalytically active substance.

15. Absorbate according to either of Claims 13 and 14,

**characterized in that** the absorbed substances are applied in proportions of 1 to 70% by weight, preferably in proportions of 3 to 60% by weight and more preferably in proportions of 5 to 50% by weight.

16. Absorbate according to either of Claims 13 and 14,
**characterized in that** the active substance or active substance mixture is applied in proportions of 1 to 20% by weight, preferably in proportions of 3 to 20% by weight and more preferably in proportions of 5 to 15% by weight.

17. Process for producing absorbates according to any of Claims 13 to 16, **characterized in that** a granular silica according to any of Claims 1 to 5 is contacted with at least one liquid selected from the group consisting of hardening agents or initiators, crosslinking agents, catalysts, active pharmaceutical ingredients and excipients, active cosmetic ingredients and excipients, cleansing and/or care compositions, flavourings, aromas and fragrances, animal feeds or animal feed additives, foods or food additives, dyes and/or pigments, amino acids, oxidizing or bleaching agents, additives with microbicidal, especially fungicidal or bactericidal, action, chemicals for agriculture and forestry, and/or a concrete admixture.

18. Use of the absorbates according to any of Claims 13 to 16 in catalytic processes.

19. Process according to Claim 18,
**characterized in that** the process is performed in a fixed bed reactor, a fluidized bed reactor, or by suspending the absorbates in a reaction mixture.

20. Use according to either of Claims 18 and 19,
**characterized in that** the absorbates comprise an enzyme as a catalyst on a granular silica according to any of Claims 1 to 5.

**Revendications**

1. Silice granulaire, ayant

    - un volume poreux par Hg (< 4 $\mu$m) de plus de 0,90 ml/g,
    - une valeur $d_{Q3=10\%}$ de plus de 400 $\mu$m avec simultanément une valeur $d_{Q3=90\%}$ de moins de 3 000 $\mu$m, le granulat étant ajusté à la fraction de particules appropriée par tamisage ou granulation avec tamisage à une granulométrie de 3 000 $\mu$m et élimination par tamisage de la fraction de fines avec une largeur de mailles de tamisage de 400 $\mu$m, et
    - un rapport entre la valeur $d_{50}$ sans l'effet d'ultrasons et la valeur $d_{50}$ après 3 min d'effet d'ultrasons < 4,00, la mesure ayant lieu sur une fraction de particules de 400 à 500 $\mu$m.

2. Silice granulaire selon la revendication 1, **caractérisée en ce qu'**elle présente un pH dans la plage allant de 5 à 8,5.

3. Silice granulaire selon les revendications 1 à 2, **caractérisée en ce qu'**elle présente un rapport entre la valeur $d_{50}$ sans l'effet d'ultrasons et la valeur $d_{50}$ après 3 min d'effet d'ultrasons de 1,00 à 3,00, de préférence de 1,00 à 2,60, de manière particulièrement préférée de 1,00 à 2,10, de manière spécialement préférée de 1,00 à 1,60 . La mesure a lieu sur une fraction de particules de 400 à 500 $\mu$m.

4. Silice granulaire selon les revendications 1 à 2, **caractérisée en ce qu'**elle présente un volume poreux par Hg (< 4 $\mu$m) de plus de 1,35 ml/g, de préférence de plus de 1,60 ml/g, de manière particulièrement préférée de plus de 1,80 ml/g, de manière spécialement préférée de plus de 1,90 ml/g.

5. Silice granulaire selon les revendications 1 à 2, **caractérisée en ce qu'**elle présente un volume poreux par Hg (< 4 $\mu$m) de 0,9 à 1,34 ml/g, de manière particulièrement préférée de 0,9 ml/g à 1,30 ml/g, de manière particulièrement préférée de 0,9 ml/g à 1,20 ml/g.

6. Procédé de fabrication de silices granulaires, comprenant les étapes suivantes :

    a) la préparation d'une silice précipitée ou pyrogénée, séchée et/ou broyée, ayant

       - une taille de particule moyenne $d_{50}$ sans traitement aux ultrasons de 0,1 à 350 $\mu$m,

- une surface BET de 30 à 800 m$^2$/g et
- un indice DBP de 140 à 400 g/100 g ;

b) l'humidification de la silice de l'étape a) selon le procédé de façonnage utilisé à une perte au séchage de 30 à 80 % en poids;

c) le façonnage de la silice de l'étape b) par extrusion, granulation, compactage ou d'autres procédés de façonnage usuels ;

d) le séchage du corps moulé de silice dans des appareils de séchage appropriés pour cela ; et

e) la granulation avec tamisage ou le tamisage du granulat à une granulométrie de 3 000 $\mu$m et l'élimination par tamisage de la fraction de fines avec une largeur de mailles de tamisage de 400 $\mu$m.

7. Procédé selon la revendication 6, **caractérisé en ce qu'**un gâteau de filtration contenant de l'eau ayant une perte au séchage de 30 à 80 % en poids est utilisé en tant que matériau de départ pour l'étape c).

8. Procédé selon la revendication 6, **caractérisé en ce que** la silice de l'étape a) est compactée et granulée à l'étape c) dans des mélangeurs intensifs rapides.

9. Procédé de fabrication de silices granulaires, comprenant les étapes suivantes :

i) la préparation d'une silice précipitée ou pyrogénée, séchée et/ou broyée, ayant une perte au séchage < 30 % en poids, et présentant

- une taille de particule moyenne d$_{50}$ sans traitement aux ultrasons de 0,1 à 350 $\mu$m,
- une surface BET de 30 à 800 m$^2$/g et
- un indice DBP de 140 à 400 g/100 g ;

ii) le façonnage de la silice de l'étape i) par compactage à sec, de préférence entre deux cylindres rotatifs, à une pression spécifique de 0,5 kN/cm de largeur de cylindre à 12 kN/cm de largeur de cylindre, pour former des produits emboutis et

iii) la granulation avec tamisage ou le tamisage du granulat à une granulométrie de 3 000 $\mu$m et l'élimination par tamisage de la fraction de fines avec une largeur de mailles de tamisage de 400 $\mu$m.

10. Procédé selon l'une quelconque des revendications 6 à 9, **caractérisé en ce que** toutes les fractions de tamisage inférieures à 400 $\mu$m sont séparées.

11. Procédé selon l'une quelconque des revendications 6 à 9, **caractérisé en ce que** les étapes de façonnage c) et ii) sont réalisées sans l'introduction de liants.

12. Utilisation des silices granulaires selon l'une quelconque des revendications 1 à 5 pour la fabrication de produits d'absorption.

13. Produits d'absorption comprenant au moins une des silices granulaires selon l'une quelconque des revendications 1 à 5.

14. Produits d'absorption selon la revendication 13, **caractérisés en ce qu'**ils contiennent au moins une substance catalytiquement active.

15. Produit d'absorption selon l'une quelconque des revendications 13 ou 14, **caractérisé en ce que** les substances absorbées sont appliquées en proportions de 1 à 70 % en poids, de préférence en proportions de 3 à 60 % en poids et de manière particulièrement préférée en proportions de 5 à 50 % en poids.

16. Produit d'absorption selon l'une quelconque des revendications 13 ou 14, **caractérisé en ce que** la substance active ou le mélange de substances actives est appliqué en proportion de 1 à 20 % en poids, de préférence en proportions de 3 à 20 % en poids et de manière particulièrement préférée en proportions de 5 à 15 % en poids.

17. Procédé de fabrication de produits d'absorption selon l'une quelconque des revendications 13 à 16, **caractérisé en ce qu'**une silice granulaire selon l'une quelconque des revendications 1 à 5 est mise en contact avec au moins un liquide choisi dans le groupe constitué par les agents de durcissement ou les initiateurs, les agents de réticulation, les catalyseurs, les agents actifs et les adjuvants pharmaceutiques, les agents actifs et les adjuvants cosmétiques, les agents de nettoyage et/ou de soin, les agents aromatisants, les arômes et les parfums, les aliments pour animaux et les additifs d'aliments pour animaux, les produits alimentaires et les additifs de produits alimentaires, les colorants et/ou les pigments, les acides aminés, les agents oxydants ou blanchissants, les additifs à effet microbicide, notamment fongicide ou bactéricide, les produits chimiques pour l'agriculture et la foresterie, et/ou un additif pour le béton.

**18.** Utilisation des produits d'absorption selon l'une quelconque des revendications 13 à 16 dans des procédés catalytiques.

**19.** Procédé selon la revendication 18, **caractérisé en ce que** le procédé est réalisé dans un réacteur à lit fixe, un réacteur à lit fluidisé ou par suspension des produits d'absorption dans un mélange réactionnel.

**20.** Utilisation selon l'une quelconque des revendications 18 ou 19, **caractérisée en ce que** les produits d'absorption contiennent une enzyme en tant que catalyseur sur une silice granulaire selon l'une quelconque des revendications 1 à 5.

Figur 1

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006002765
- EP 0984772 B1
- EP 0966207 B1
- DE 102008035867 A1
- EP 0937755 A1